# EUROPEAN PATENT SPECIFICATION

(11) **EP 0 543 021 B1**
(45) Date of publication and mention of the grant of the patent: **28.04.1999**
(21) Application number: 92911224.1
(22) Date of filing: 10.06.1992
(51) Int. Cl.: C12N 11/00, C12P 13/04, C12N 11/08, C12N 9/96

(54) **IMMOBILIZED ENZYME PREPARATION AND PRODUCTION OF D-ALPHA-AMINO ACID**
IMMOBILISIERTE ENZYMPREPARATION UND HERSTELLUNG VON D-ALPHA-AMINOSÄURE
PREPARATION ENZYMATIQUE IMMOBILISEE ET PRODUCTION DE D-ALPHA-AMINOACIDE

(30) Priority: 12.06.1991 JP 140051/91
(43) Date of publication of application: 26.05.1993
(73) Proprietor: KANEGAFUCHI KAGAKU KOGYO KABUSHIKI KAISHA, Kita-ku, Osaka-shi, Osaka 530 (JP)
(72) Inventor: YAMADA, Yukio, Kakogawa-shi, Hyogo 675 (JP); NANBA, Hirokazu, Takasago-shi, Hyogo 676 (JP); TAKANO, Masayuki, Akashi-shi, Hyogo 674 (JP); IKENAKA, Yasuhiro, Akashi-shi, Hyogo 673 (JP); TAKAHASHI, Satomi, Kobe-shi, Hyogo 655 (JP); OKUBO, Kazuma, Kakogawa-shi, Hyogo 675-01 (JP); YAMADA, Kazuhiko, Akashi-shi, Hyogo 674 (JP); HIRAISHI, Yoshiro, Himeji-shi, Hyogo 679-21 (JP)
(74) Representative: Stuart, Ian Alexander
(86) International application number: JP9200739
(87) International publication number: WO9222643

(56) References cited:
- EP-A- 0 515 698
- JP-B-63 020 520
- CHEMICAL & ENGINEERING NEWS vol. 53, no. 33 , 18 August 1975 pages 22 - 40 K.J SKINNER 'enzymes technology'
- ADVANCES IN BIOCHEMICAL ENGINEERING vol. 12 , 1979 , NEW YORK pages 42 - 118 R.SCHMID 'Stabilized soluble enzymes'
- THE WORLD BIOTECH REPORT 1984 vol. 1 , 1984 pages 379 - 390 P. MONSAN 'Stabilization of enzyme activity'

## Description

The present invention relates to an immobilized enzyme preparation and a process for producing D-α-amino acids using the same. In particular, the immobilized enzyme preparation of the present invention serves for the production of D-α-amino acids which are available as intermediate materials of antibiotics, such as D-(p-hydroxyphenyl)glycine used in the production of antibiotic amoxicillin.

It is known from JP-B 57-18793, JP-B 63-20520, JP-B 1-48758, etc., that there exist some enzymes (hereinafter referred to as "decarbamylases") capable of converting N-carbamyl-D-α-amino acid derivatives into D-α-amino acids.

With respect to numerous other enzymes, it is known that the use of these enzymes or enzyme-producing cells in insolubilized or immobilized form makes it possible to prepare an enzyme reaction mixture having only a few impurities, which results in an easy recovery of the product, and also makes it possible to attain the repeated use of these enzymes, which is advantageous from the view point of cost recovery. Moreover, there has been obtained a good effect by the use in the production on an industrial scale.

In the case of a decarbamylase, however, the possibility of using this enzyme as an immobilized enzyme is generally suggested as a mode of use only in JP-B 63-20520, although there are some examples of using this enzyme in the form of whole cells, toluene-treated bacterial cells, acetone-treated powder of cells or cell-free extracts.

US-A-4 312 948 discloses the use of Agrobacterium spp and enzyme preparations thereof for producing D-aminoacids from N-carbamoyl derivatives. Immobilisation of the enzymes is said to offer unspecified advantages; this is not exemplified.

These examples of using a decarbamylase have a problem of how to maintain its enzyme activity. In particular, the enzyme preparations lose activity on repeated use, and they cannot be used on a commercial basis.

The present invention is to obtain an immobilized enzyme preparation of decarbamylases with stability sufficient to withstand the repeated use and attempts to use it in the production on an industrial scale.

Under these circumstances, the present inventors have intensively studied. As the result, they have found that an antioxidant is effective for the stabilization of specified decarbamylases and that a repeatedly usable immobilized preparation of decarbamylases can be obtained by bringing the carbamylases into contact with a solid support in the presence thereof, thereby completing the present invention.

In one aspect the invention provides an immobilized enzyme preparation which comprises an enzyme capable of producing D-α-amino acids by hydrolysis of N-carbamyl-D-α-amino acids, and a support for immobilization, characterised in that said enzyme is one produced by a microorganism selected from Pseudomonas sp/ KNK 003A (FERM BP-3181), Pseudomonas sp. KNK 505 (FERM BP-3182), or Escherichia coli JM109 pPD304 (FERM BP-3183); and in that said preparation includes an antioxidant; and is capable of repeated use 30 times or more.

In further aspects the invention provides a process for producing an immobilized enzyme preparation, in which an enzyme as defined above is brought into contact with a support for immobilization in the presence of an antioxidant; and a process for producing D-α-amino acids, in which the said immobilized enzyme preparation is allowed to act on N-carbamyl-D-α-amino acids.

The decarbamylase used in the present invention may be one having a stereospecific action on D-amino acids or one having an action on both of D- and L-amino acids. Depending on the mode of use, enzymes specific to D-amino acids are usually useful in many cases.

In the present invention, the said decarbamylase may be present in the immobilized enzyme preparation, as a purified enzyme, a partially-purified enzyme or a crude enzyme, or in some cases, as it is in the form of bacterial cells. If only this enzyme is under the conditions that it can exhibit the decarbamyl activity, there is no need for take care to its existence form and contaminants.

As the antioxidant, dithiothreitol, 2-mercaptoethanol, L-cysteine hydrochloride, cysteamine hydrochloride, dithioerythritol, mixtures of dithiothreitol and dithioerythritol, or reduced-type glutathione can be used.

The support used for immobilization may vary depending upon the mode of decarbamylase immobilization; in cases where crude enzyme solutions such as cell-free extracts or partially-purified enzyme solutions obtained by fractionation with ammonium sulfate are subjected to immobilization, a polymer support having ion exchange groups or covalent-bonding groups can be used.

As the polymer support having ion exchange groups, an anionic exchange resin such as a series of Duolite (registered trade mark) A and Amberlite IRA (registered trade mark), having primary, secondary, tertiary and quaternary amines as exchange groups, or a polystyrene resin having diethanol-type functional groups, such as Diaion EX, can be used.

As those having covalent-bonding groups, a substituted polymethacrylate polymer having aldehydes as bonding groups, or high-density alumina covered with a polyethyleneimine/glutaraldehyde composite can be used.

Moreover, for immobilization of whole cells such as viable bacterial cells or dried bacterial cells, a polymer such as polyacrylamide, polyurethane and calcium alginate, or a porous material such as pumice and alumina, can be used.

The immobilized enzyme preparation of the present invention can be produced as follows.

First, a culture of microorganisms is prepared, and bacterial cells are collected therefrom. Then, a cell-free extract is prepared by ultrasonic disruption, mechanical disruption (e.g., homogenizer), enzyme treatment or the like. In this case, an antioxidant is allowed to exist at a concentration of 0.1 to 20 mM, usually about 5 mM.

For the partial purification of an enzyme from the cell-free extract, any means usually used by those skilled in the art is employed. For example, nucleic acids are precipitated by addition of protamine sulfate in a 1/5 to 1/10 quantity to the amount of proteins, and after heating at 500 to 60°C for about 20 minutes, heat-unstable proteins are precipitated by cooling to 4°C, after which the nucleic acids and the heat unstable proteins are removed by centrifugation. Further, fractionation with ammonium sulfate is conducted, if necessary, to obtain an enzyme solution to be brought into contact with a support. During this step, an antioxidant is allowed to exist at a concentration of 0.1 to 20 mM, usually about 5 mM.

The support whose exchange groups has been activated with saline and then equilibrated in a buffer containing 0.1 to 20 mM, usually about 5 mM, of antioxidant is used. The support and the enzyme solution are brought into contact with each other in the presence of an antioxidant at a concentration of 0.1 to 20 mM, usually about 5 mM, followed by stirring at 4° to 30°C, preferably 25°C. After the amount of enzyme adsorbed reaches the desired amount (usually after 8 to 48 hours), the support is removed by filtration and treated with a cross-linking agent to attain the stability of enzymes by insolubilization. As the cross-linking agent, for example, those known in the art can be used, such as not greater than 1% (preferably 0.2%) glutaraldehyde. It is also preferred that an antioxidant is allowed to exist at a concentration of 0.1 to 20 mM during this step. The cross-linked, immobilized enzyme preparation is washed with distilled water or a buffer (preferably containing an antioxidant), and stored under moisturized conditions in a sealed vessel at low temperatures (about 4°C).

It is preferred that all procedures are conducted, if possible, in an inert gas atmosphere, e.g. under nitrogen. The amount of enzyme to be loaded, although it may vary depending upon the degree of purification of this enzyme, is usually in the range of 10 to 80 units/g of support (wherein one unit of decarbamylase refers to the amount of enzyme required to convert 1 µmol of N-carbamyl-D-(p-hydroxyphenyl)glycine into D-(p-hydroxyphenyl)glycine within one minute). It is preferred that an antioxidant is allowed to exist in the immobilized enzyme preparation at an amount of 5 to 10 mg/g of the preparation.

Then, the following will describe the process for producing D-α-amino acids from N-carbamyl-D-α-amino acids by use of an immobilized preparation of a decarbamylase.

The reaction is conducted in the presence of an antioxidant, and it can be expressed by the reaction scheme: 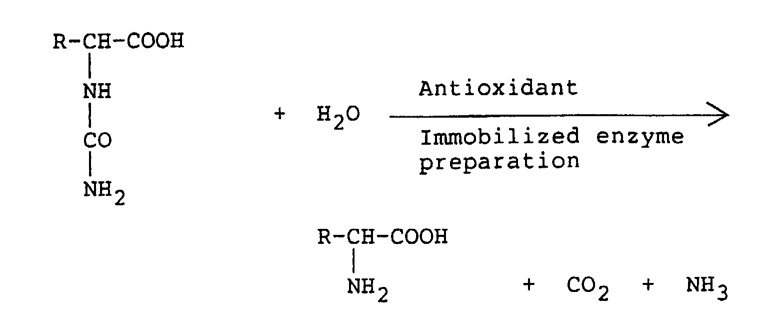 wherein R is C₁-C₄ alkyl optionally substituted with a group such as hydroxy, alkylthio, carboxy, amino or amide; C₇-C₈ aralkyl (e.g., benzyl, phenethyl) having an aromatic ring optionally substituted with a group such as hydroxy; or phenyl optionally substituted with hydroxy or halogen.

The amount of antioxidant is in the range of 0.1 to 20 mM, preferably 2.0 to 5.0 mM.

The concentration of N-carbamyl-D-α-amino acid as a reaction substrate is selected in the range of 5 to 25 w/v%, and preferably used at 15 w/v%. The amount of immobilized enzyme preparation used is selected in the range of 10 to 20 units per substrate, and preferably used at 15 units/g of substrate, for those containing 10 to 80 units/g of support. The reaction is conducted, while controlling the pH in the range of pH 6.5 to 8.0, usually to pH 7.0. The temperature, although it may vary depending upon the kind of enzyme, is generally in the range of 30° to 60°C; in particular, higher temperatures can be employed for heat resistant enzymes. The reaction is conducted by a column technique or by suspension in a reaction vessel; in the latter case, batch reaction is usually conducted for a period of reaction time in the range of about 6 to 48 hours per batch. The immobilized enzyme preparation of the present invention is quite stable; for example, it exhibited almost no decrease in its activity, even if used 15 times or more, and repeated use 30 times or more was possible.

Preferably, the atmosphere in the reaction vessel is replaced by an inert gas and remains containing no oxygen.

The invention will be explained in more detail by way of the following Examples, which are only illustrative and not to be construed to limit the scope thereof.

### Example 1

The decarbamylase was produced by bacterial fermentation using the following procedures.

| Medium ingredients: | |
|---|---|
| Sucrose | 300 g |
| Urea | 25.5 g |
| KH₂PO₄ | 30 g |
| Na₂HPO₄ | 30 g |
| HgSO₄·7H₂O | 15 g |
| MnCl₂·4H₂O | 150 mg |
| Yeast extract | 75 g |
| N-carbamyl-D-phenylglycine | 15 g |
| Anti-foaming agent | 15 g |

In this medium, Pseudomonas sp. KNK003A (FERM BP-3181) was grown, and the cells harvested from 9 liters of fermentation broth by centrifugation. These cells were resuspended in 300 ml of 0.2 M Tris/HCl buffer (pH 7.0), and dithiothreitol was added to give a concentration of 5 mM, followed by ultrasonic treatment to discharge a decarbamylase into the solution. Then, 20 ml of 3% aqueous protamine sulfate was gradually added thereto with stirring, thereby precipitating nucleic acids. The enzyme solution was heat treated at 65°C for 20 minutes, and then cooled to 4°C. After storage overnight at 4°C, the nucleic acids, as well as proteins, were precipitated, and the cell debris was removed by centrifugation. Then, 100 ml of this partially-purified enzyme solution after the heat treatment (activity: 0.06 units/ml) was added to 10 g of the resin Duolite A568. This mixture was stirred at 25°C for 24 hours, thereby allowing the decarbamylase to be adsorbed on the support. The enzyme-resin composite was removed by filtration, and cross-linked by moderately stirring in 0.2% glutaraldehyde solution (pH 7.0) containing 5 mM dithiothreitol for 1 hour. The mixture was washed three times with distilled water, and finally washed with 0.2 M Tris/HCl buffer (pH 7.0). The specific activity thereof was 0.38 units/g of resin, and the activity in the case where N-carbamyl-D-alanine was used as a substrate was 4.2 units/g.

### Example 2

Using 5 g of the enzyme-resin produced by the process as described in Example 1, 2 g of N-carbamyl-D-alanine was hydrolyzed in 40 ml of distilled water containing 0.5 mM dithiothreitol. The hydrolysis was conducted at 45°C, at pH 6.7 to 7.0, for 24 hours, while blowing N₂ into this mixture to exclude oxygen therefrom. The yield of D-alanine after completion of the reaction was determined to be 96.5%.

Then, the hydrolysis of 5% N-carbamyl-D-alanine was conducted 35 times to give an average yield of 96.1%.

### Example 3

First, 1 g of the enzyme-resin produced by the process as described in Example 1 was charged into a column, and 2% N-carbamyl-D-(p-hydroxyphenyl)glycine solution containing 1 mM antioxidant was applied to the above column for continuous hydrolysis. As the antioxidant, dithiothreitol, L-cysteine hydrochloride or cysteamine hydrochloride was used, and the continuous reaction was conducted at an application speed of 1 ml/min., at 40°C under N₂ sealing for 4 days.

With the use of these antioxidants, the degree of enzyme deactivation for 4 days was suppressed to 30% to 35%, whereas the enzyme deactivation of 80% or more was found when no antioxidant was used.

| Antioxidant | Ratio of remaining enzyme activity (%) after | | | |
|---|---|---|---|---|
| | 1 day | 2 days | 3 days | 4 days |
| Dithiothreitol | 80 | 70 | 68 | 65 |
| L-cysteine hydrochloride | 78 | 71 | 68 | 66 |
| Cysteamine hydrochloride | 78 | 75 | 73 | 68 |
| Dithioerythritol | 78 | 72 | 68 | - |
| No addition | 50 | 29 | 20 | 14 |

As described above, according to the present invention, there is provided an immobilized preparation of decarbamylases capable of producing D-α-amino acids which are important as intermediate materials of antibiotics and the like. The use of this preparation makes possible the repeated use at 30 times or more, which gives the expectation of using it in the production on an industrial scale.

## Claims

1. An immobilized enzyme preparation which comprises an enzyme capable of producing D-α-amino acids by hydrolysis of N-carbamyl-D-α-amino acids, and a support for immobilization, characterised in that said enzyme is one produced by a microorganism selected from Pseudomonas sp/ KNK 003A (FERM BP-3181), Pseudomonas sp. KNK 505 (FERM BP-3182), or Escherichia coli JM109 pPD304 (FERM BP-3183); and in that said preparation includes an antioxidant; and is capable of repeated use 30 times or more.

2. An immobilized enzyme preparation according to claim 1, wherein the support for immobilization is an anion exchange resin.

3. An immobilized enzyme preparation accordingto claim 2, wherein the anion exchange resin is a phenol-type ion exchange resin having an amine as an exchange group.

4. An immobilized enzyme preparation according to claim 1, wherein the antioxidant is dithiothreitol, 2-mercaptoethanol, L-cysteine hydrochloride, cysteamine hydrochloride, dithioerythritol, mixtures of dithiothreitol and dithioerythritol, or reduced-type glutathione.

5. A process for producing an immobilized enzyme preparation, wherein an enzyme capable of producing D-α-amino acids by hydrolysis of N-carbamyl-D-α-amino acids is brought into contact with a support for immobilization in the presence of an antioxidant, characterised in that said enzyme is one produced by a microorganism selected from Pseudomonas sp/ KNK 003A (FERM BP-3181), Pseudomonas sp. KNK 505 (FERM BP-3182), or Escherichia coli JM109 pPD304 (FERM BP-3183).

6. A production process according to claim 5, wherein the support for immobilization is equilibrated in a buffer containing the antioxidant and thereafter brought into contact with the enzyme.

7. A process for producing a D-α-amino acid, characterized in that an immobilized enzyme preparation according to any one of claims 1 to 4 is allowed to act on an N-carbamyl-D-α-amino acid.

8. A process for producing a D-α-amino acid according to claim 7, wherein the immobilized enzyme preparation is allowed to act with the addition of an antioxidant to the reaction system.

9. A production process according to claim 7 or 8 wherein the immobilized enzyme preparation is repeatedly used.

## Patentansprüche

1. Immobilisiertes Enzympräparat, das ein Enzym, welches zur Erzeugung von D-α-Aminosäuren durch Hydrolyse von N-Carbamyl-D-α-aminosäuren fähig ist, sowie einen Träger zur Immobilisierung umfaßt, dadurch gekennzeichnet, daß es sich bei dem Enzym um eines handelt, das von einem Mikroorganismus, ausgewählt aus Pseudomonas sp/KNK 003A (FERM BP-3181), Pseudomonas sp. KNK 505 (FERM BP-3182) oder Escherichia coli JM109 pPD304 (FERM BP-3183), produziert wird; und daß das Präparat ein Antioxidans umfaßt und dreißig Mal oder öfter wiederverwendet werden kann.

2. Immobilisiertes Enzympräparat nach Anspruch 1, worin der Träger zur Immobilisierung ein Anionenaustauschharz ist.

3. Immobilisiertes Enzympräparat nach Anspruch 2, worin das Anionenaustauschharz ein Ionenaustauschharz vom Phenoltyp mit einem Amin als Austauschgruppe ist.

4. Immobilisiertes Enzympräparat nach Anspruch 1, worin das Antioxidans Dithiothreitol, 2-Mercaptoethanol, L-Cysteinhydrochlorid, Cysteaminhydrochlorid, Dithioerythrit, Gemische aus Dithiothreitol und Dithioerythrit, oder Glutathion vom reduzierten Typ ist.

5. Verfahren zur Herstellung eines immobilisierten Enzympräparats, worin ein Enzym, das zur Erzeugung von D-α-Aminosäuren durch Hydrolyse von N-Carbamyl-D-α-aminosäuren fähig ist, mit einem Träger zur Immobilisierung in Gegenwart eines Antioxidans in Kontakt gebracht wird, dadurch gekennzeichnet, daß es sich bei dem Enzym um eines handelt, das von einem Mikroorganismus, ausgewählt aus Pseudomonas sp/KNK 003A (FERM BP-3181), Pseudomonas sp. KNK 505 (FERM BP-3182) oder Escherichia coli JM109 pPD304 (FERM BP-3183), produziert wird.

6. Herstellungsverfahren nach Anspruch 5, worin der Träger zur Immobilisierung in einem Puffer äquilibriert wird, der das Antioxidans enthält, und daraufhin mit dem Enzym in Kontakt gebracht wird.

7. Verfahren zur Herstellung einer D-α-Aminosäure, dadurch gekennzeichnet, daß ein immobilisiertes Enzympräparat nach einem der Ansprüche 1 bis 4 auf eine N-Carbamyl-D-α-aminosäure einwirken gelassen wird.

8. Verfahren zur Herstellung einer D-α-Aminosäure nach Anspruch 7, worin das immobilisierte Enzympräparat gleichzeitig mit der Zugabe eines Antioxidans zum Reaktionssystem einwirken gelassen wird.

9. Herstellungsverfahren nach Anspruch 7 oder 8, worin das immobilisierte Enzympräparat wiederholt verwendet wird.

## Revendications

1. Préparation d'enzyme immobilisée qui comprend une enzyme capable de produire des D-α-aminoacides par hydrolyse de N-carbamyl-D-α-aminoacides, et un support pour l'immobilisation, caractérisée en ce que ladite enzyme est une enzyme produite par un micro-organisme sélectionné parmi Pseudomonas sp/KNK 003A (FERM BP-3181), Pseudomonas sp. KNK 505 (FERM BP-3182) ou Escherichia coli JM109 pPD304 (FERM BP-3183), et en ce que ladite préparation comprend un antioxydant, et peut supporter des utilisations répétées 30 fois ou plus.

2. Préparation d'enzyme immobilisée selon la revendication 1, dans laquelle le support d'immobilisation est une résine échangeuse d'anions.

3. Préparation d'enzyme immobilisée selon la revendication 2, dans laquelle la résine échangeuse d'anions est une résine échangeuse d'anions du type phénol ayant une amine en tant que groupe d'échange.

4. Préparation d'enzyme immobilisée selon la revendication 1, dans laquelle l'antioxydant est le dithiothréitol, le 2-mercaptoéthanol, le chlorhydrate de L-cystéine, le chlorhydrate de cystéamine, le dithioérythritol, des mélanges de dithiothréitol et de dithioérythritol, ou le glutathion de type réduit.

5. Procédé de production d'une préparation d'enzyme immobilisée dans lequel une enzyme capable de produire des D-α-aminoacides par hydrolyse de N-carbamyl-D-α-aminoacides est mise en contact avec un support d'immobilisation en présence d'un antioxydant, caractérisé en ce que ladite enzyme est une enzyme produite par un micro-organisme sélectionné parmi Pseudomonas sp/KNK 003A (FERM BP-3181), Pseudomonas sp. KNK 505 (FERM BP-3182), Escherichia coli JM109 pPD304 (FERM BP-3183).

6. Procédé de production selon la revendication 5, dans lequel le support d'immobilisation est équilibré dans un tampon contenant l'antioxydant et mis ensuite en contact avec l'enzyme.

7. Procédé de production d'un D-α-aminoacide, caractérisé en ce qu'on permet à une préparation d'enzyme immobilisée selon l'une quelconque des revendications 1 à 4 d'agir sur un N-carbamyl-D-α-aminoacide.

8. Procédé de production d'un D-α-aminoacide selon la revendication 7, dans lequel on permet à la préparation d'enzyme immobilisée d'agir avec l'addition d'un antioxydant au système réactionnel.

9. Procédé de production selon la revendication 7 ou 8, dans lequel la préparation d'enzyme immobilisée est utilisée de façon répétée.
